# EUROPEAN PATENT SPECIFICATION

(11) **EP 1 398 187 B1**
(45) Date of publication and mention of the grant of the patent: **28.04.2004**
(21) Application number: 02425782.6
(22) Date of filing: 18.12.2002
(51) Int. Cl.: B60H 3/00, A61L 9/12

(54) **A device for scenting an air flow intended to be introduced in the passenger compartment of a vehicle**
Duftspendevorrichtung für einem Luftstrom, der dem Fahrgastraum zugeführt wird
Appareil permettant de parfumer un flux d'air destiné au compartiment passager d'un véhicule

(43) Date of publication of application: 17.03.2004
(73) Proprietor: DENSO THERMAL SYSTEMS S.p.A., 10046 Poirino (Torino) (IT)
(72) Inventor: Gataleta, Raffaele, 10155 Torino (IT)
(74) Representative: Marchitelli, Mauro

(56) References cited:
- EP-A- 0 483 848
- FR-A- 2 821 022
- GB-A- 2 249 958
- US-A- 5 314 669

## Description

The present invention relates to a device for scenting an air flow that is to be introduced into the passenger compartment of a vehicle.

Document GB 2 249 958 A discloses such a device.

The air-treatment assembly of a vehicle assumes increasingly greater importance from the point of view of the level of quality of a vehicle. In addition to the traditional functions of heating, conditioning and de-humidification of the flow of air, it would be desirable to increase further the quality of the flow of air introduced into the passenger compartment of the vehicle by the addition of aromas or perfumes. Normally, in order to perfume the passenger compartment of a vehicle recourse is had to the use of scenting products located in the passenger compartment. This solution is not, however, acceptable for high-quality vehicles.

The purpose of the present invention is to provide a device which can be associated to, or integrated in, a heating and/or air-conditioning assembly, for scenting a flow of air upstream of its introduction into the passenger compartment. A further purpose of the present invention is to provide a device for scenting a flow of air which will enable ease of regulation of the degree of scenting of the flow of air and possible selection of the desired fragrance.

According to the present invention, the aforesaid purposes are achieved by a device presenting the characteristics forming the subject of the ensuing claims.

The present invention will now be described in detail with reference to the attached drawings, which are provided purely by way of non-limiting example and in which:

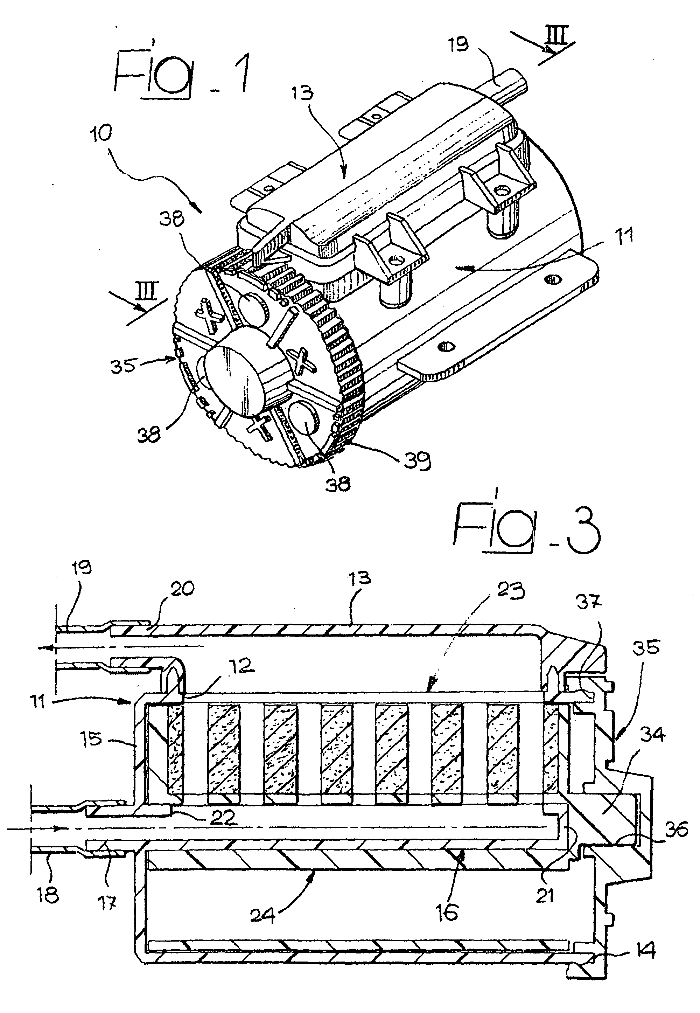

- Figure 1 is a perspective view of a device according to the present invention;

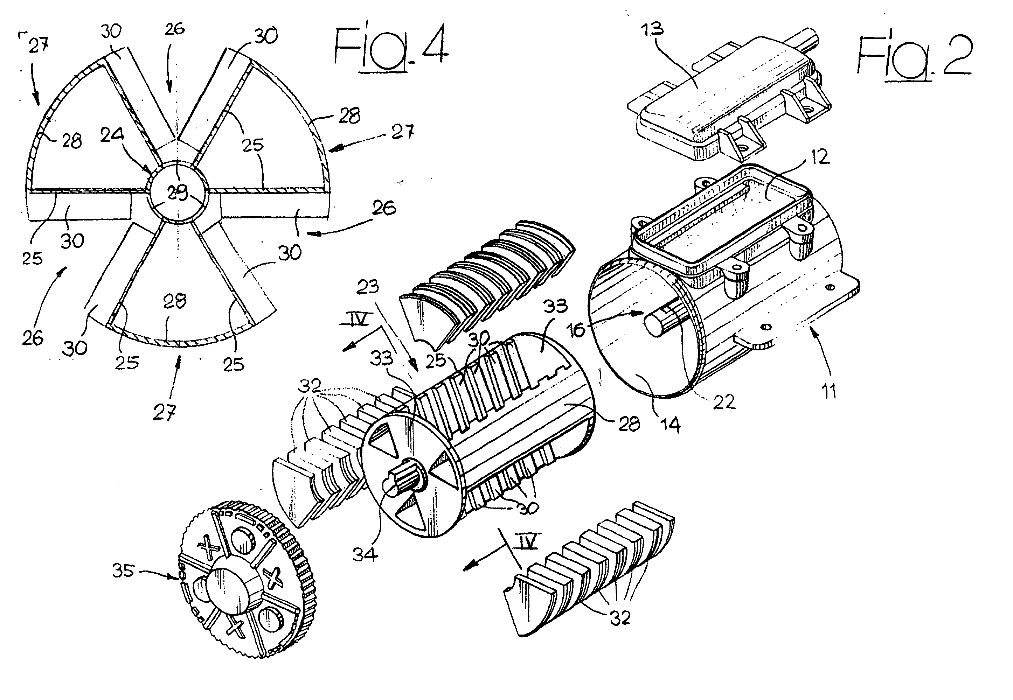

- Figure 2 is an exploded perspective view of the device of Figure 1; and
- Figure 3 is a cross section along the line III of Figure 1.

With reference to the figures, the number 10 designates a device for scenting an air flow that is to be introduced into the passenger compartment of a vehicle. The device 10 comprises a cylindrical casing 11 designed to be fixed to the vehicle. The casing 11 is preferably made of a moulded plastic material and has a side opening 12 closed by a lid 13 fixed in a sealed way along the edge of the opening 12. The cylindrical casing 11 has a front opening 14 and, on the opposite side, a radial bottom wall 15. A hollow shaft 16 extends coaxially inside the casing 11. The hollow shaft 16 is fixed with respect to the casing 11. Preferably, the hollow shaft 16 is formed integrally with the radial bottom wall 15 of the casing 11 and has an integral projecting portion 17, which extends on the outer side of the bottom wall 15. A first flexible tube 18 is connected to the projecting portion 17. A second flexible tube 19 is connected to a tubular connecting portion 20 formed integrally with the lid 13.

The hollow shaft 16 has a closed front end 21 opposite to the projecting portion 17 and a longitudinal through groove 22 set facing the side opening 12 of the casing 11.

The device 10 comprises a cylindrical body 23 housed inside the casing 11. The cylindrical body 23 is preferably made of moulded plastic material and comprises a central tubular shaft 24, which is mounted so that it can turn on the external surface of the hollow shaft 16 of the casing 11. The cylindrical body 23 comprises a plurality of radial walls 25, which pass through the axis of the tubular shaft 24 and divide the cylindrical body 23 into a plurality of sectors 26, 27. The radial walls 25 are alternately connected to one another by circumferential walls 28 so as to form alternately open sectors 26 and closed sectors 27. In the example illustrated in the figures, six radial walls 25 are provided, set at an angular distance of 60° apart from one another and set so as to form three open sectors 26 and three closed sectors 27. The tubular shaft 24 of the cylindrical body 23 has openings 29 at the open sectors 26.

The radial walls 25 of the cylindrical body 23 have, at the open sectors 26, a plurality of projecting ribbings 30, which define a plurality of seats designed to receive respective tablets 32 of scenting material. The tablets 32 are set at a distance apart from one another along the longitudinal axis of the cylindrical body 23.

The cylindrical body 23 has two radial end walls 33, which delimit laterally the open sectors 26. The tubular shaft 24 of the cylindrical body 23 has a projecting portion 34, which extends beyond one of the radial end walls 33.

The device 10 comprises a control member 35, which enables adjustment of the angular position of the cylindrical body 23 inside the casing 11. The control member 35 is built in the form of a rotatable lid, which closes the front opening 14 of the casing 11. The control member 35 has a seat 36, which engages the projecting portion 34 of the tubular shaft 24, rendering the control member 35 fixed, in rotation, to the cylindrical body 23. The control member 35 moreover has an annular groove 37, which engages the end edge of the casing 11.

In use, the first flexible tube 18 is connected to the assembly for treating the flow of air of the vehicle downstream of the fan assembly. A part of the flow of air produced by the fan assembly is sent to the scenting device 10. This part of the flow enters the tubular shaft 24 of the rotating body 23 and laps the radial walls of the tablets 32. The flow of air is collected in the chamber formed by the lid 13 and exits from the device 10 through the second flexible tube 19.

The second flexible tube 19 carries back the flow of scented air into the air-treatment-and-distribution assembly, for example upstream of the area of distribution of the flow into the passenger compartment of the vehicle, directly in the passenger compartment or in the air-distribution ducts present in the dashboard. By rotating the control member 35, it is possible to select the type of scenting fragrance, cut out completely perfuming of the flow of air, or else adjust the intensity of scenting. The control member 35 may be provided on its external surface with cylindrical elements 38 of different colours, which indicate the type of fragrance of the tablets contained in the corresponding sector 26. The blind sectors may be marked by a different visual indication (for example, an "X"). The control member 35 may be provided with a toothing or knurling 39, which can be gripped manually for controlling its rotation. The device 10 could be remotely controlled by the user by means of an actuator. Of course, the device according to the present invention may undergo numerous variations. For example, the number of the sectors containing the scenting tablets may be varied according to the need. Also the direction of the flow through the device 10 could vary with respect to a what is illustrated herein.

## Claims

1. A device for scenting an air flow that is to be introduced into the passenger compartment of a vehicle, **characterized in that** it comprises:
- a casing (11) having an inlet (17) and an outlet (20) for a flow of air;
- a cylindrical body (23) housed in a rotatable way inside the casing (11) and divided into a plurality of sectors (26, 27), a first set of sectors (26) being open and being provided with seats designed to receive tablets of a scenting product (32) and a second set of sectors (27) being closed; and
- a control member (35) designed to vary the angular position of the cylindrical body (23) with respect to the casing (11).

2. The device according to Claim 1, **characterized in that** the control member (35) comprises a lid mounted so that it can turn on an open circular edge (14) of the casing (11) and fixed, in rotation, to said cylindrical body (23).

3. The device according to Claim 1, **characterized in that** the cylindrical body (23) comprises a plurality of radial walls (25), which divide the cylindrical body (23) into said sectors (26, 27).

4. The device according to Claim 3, **characterized in that** said radial walls (25) are alternately connected to one another by circumferential walls (28) defining said closed sectors (27).

5. The device according to Claim 3, **characterized in that** said radial walls (25) are provided with ribbings (30) set at a distance apart from one another along the axis of the cylindrical body (23) and forming seats set at a distance apart from one another in an axial direction, designed to receive tablets (32) having the shape of sectors of a disk.

6. The device according to Claim 1, **characterized in that** the casing (11) has an opening (12) closed by a lid (13) defining a chamber connected to said outlet (20).

7. The device according to Claim 1, **characterized in that** said cylindrical body (23) comprises a central hollow shaft (24) provided with openings (29) at said open sectors (26).

8. The device according to Claim 5, **characterized in that** the casing (11) comprises a tubular shaft (16) connected to said inlet (17).

9. The device according to Claim 6, **characterized in that** said hollow shaft (24) of the cylindrical body (23) is mounted so that it can turn on the external surface of said tubular shaft (16).

## Patentansprüche

1. Vorrichtung zum Parfümieren eines Luftstromes, der in einen Fahrgastraum eines Fahrzeugs eingeführt werden soll, **dadurch gekennzeichnet, dass** sie umfasst:
- ein Gehäuse (11), welches einen Einlass (17) und einen Auslass (20) für einen Strom von Luft aufweist;
- einen zylindrischen Körper (23), der auf drehbare Weise im Inneren des Gehäuses (11) untergebracht ist und in eine Vielzahl von,Sektoren (26, 27) unterteilt ist, einen ersten Satz von Sektoren (26), die offen sind und mit Sitzen versehen sind, die konstruiert sind, um Tabletten eines parfümierenden Produktes (32) aufzunehmen und einen zweiten Satz von Sektoren (27), die geschlossen sind; und
- ein Steuerelement (35), welches konstruiert ist, um die Winkelposition des zylindrischen Körpers (23) hinsichtlich des Gehäuses (11) zu variieren.

2. Vorrichtung gemäß Anspruch 1, **dadurch gekennzeichnet, dass** das Steuerelement (35) einen Deckel umfasst, der montiert ist, so dass er auf eine offene kreisförmige Kante (14) des Gehäuses (11) drehen kann und beim Drehen an dem zylindrischen Körper (23) fixiert ist.

3. Vorrichtung gemäß Anspruch 1, **dadurch gekennzeichnet, dass** der zylindrische Körper (23) eine Vielzahl von radialen Wänden (25) umfasst, welche den zylindrischen Körper (23) in die Sektoren (26, 27) unterteilen.

4. Vorrichtung gemäß Anspruch 3, **dadurch gekennzeichnet, dass** die radialen Wände (25) abwechselnd miteinander verbunden sind durch umfängliche Wände (28), die die geschlossenen Sektoren (27) definieren.

5. Vorrichtung gemäß Anspruch 3, **dadurch gekennzeichnet, dass** die radialen Wände (25) mit Verrippungen (30) versehen sind, die in einem Abstand voneinander entlang der Achse des zylindrischen Körpers (23) fixiert sind und Sitze bilden, die in einem Abstand von einander in einer axialen Richtung fixiert sind, die konstruiert sind, um Tabletten (32) aufzunehmen, die die Form von Sektoren einer Scheibe aufweisen.

6. Vorrichtung gemäß Anspruch 1, **dadurch gekennzeichnet, dass** das Gehäuse (11) eine Öffnung (12) aufweist, das durch einen Deckel (13) geschlossen ist, welches eine Kammer definiert, die mit dem Auslass (20) verbunden ist.

7. Vorrichtung gemäß Anspruch 1, **dadurch gekennzeichnet, dass** der zylindrische Körper (23) eine zentrale Hohlwelle (24) umfasst, die mit Öffnungen (29) an den offenen Sektoren (26) versehen ist.

8. Vorrichtung gemäß Anspruch 5, **dadurch gekennzeichnet, dass** das Gehäuse (11) eine röhrenförmige Welle (16) umfasst, die mit dem Einlass (17) verbunden ist.

9. Vorrichtung gemäß Anspruch 6, **dadurch gekennzeichnet, dass** die Hohlwelle (24) des zylindrischen Körpers (23) derartig montiert ist, dass sie auf der äußeren Oberfläche der röhrenförmigen Welle (16) drehen kann.

## Revendications

1. Dispositif destiné à parfumer un flux d'air qui est introduit dans le compartiment passager d'un véhicule, **caractérisé en ce qu'**il comprend :
- un boîtier (11) présentant une admission (17) et une évacuation (20) pour une circulation d'air ;
- un corps cylindrique (23) logé de manière rotative à l'intérieur du boîtier (11) et divisé en une pluralité de secteurs (26, 27), un premier ensemble de secteurs (26) étant ouvert et étant pourvu de sièges conçus pour recevoir des tablettes de produit parfumant (32) et un deuxième ensemble de secteurs (27) étant fermé ; et
- un élément de commande (35) conçu pour modifier la position angulaire du corps cylindrique (23) par rapport au boîtier (11).

2. Dispositif selon la revendication 1, **caractérisé en ce que** l'élément de commande (35) comprend un couvercle monté de sorte qu'il puisse tourner sur un bord circulaire ouvert (14) du boîtier (11) et fixé, de manière rotative, audit corps cylindrique (23).

3. Dispositif selon la revendication 1, **caractérisé en ce que** le corps circulaire (23) comprend une pluralité de parois radiales (25) qui divise le corps cylindrique (23) en lesdits secteurs (26, 27).

4. Dispositif selon la revendication 3, **caractérisé en ce que** lesdites parois radiales (25) sont reliées alternativement l'une à l'autre par les parois circulaires (28) définissant lesdits secteurs fermés (27).

5. Dispositif selon la revendication 3, **caractérisé en ce que** lesdites parois radiales (25) sont pourvues avec des nervures (30) placées à une certaine distante les unes des autres le long de l'axe du corps cylindrique (23) et constituant des sièges établis à une distance l'un de l'autre dans la direction axiale et conçus pour recevoir des pastilles (32) ayant la forme des secteurs d'un disque.

6. Dispositif selon la revendication 1, **caractérisé en ce que** le boîtier (11) présente une ouverture (12) fermée par un couvercle (13) définissant une chambre reliée à ladite évacuation (20).

7. Dispositif selon la revendication 1, **caractérisé en ce que** ledit corps cylindrique (23) comprend un arbre creux central (24) pourvu d'ouvertures (29) auxdits secteurs ouverts (26).

8. Dispositif selon la revendication 5, **caractérisé en ce que** le boîtier (11) comprend un arbre tubulaire (16) relié à ladite admission (17).

9. Dispositif selon la revendication 6, **caractérisé en ce que** ledit arbre creux (24) du corps cylindrique (23) est monté de sorte qu'il puisse tourner sur la surface extérieure dudit arbre tubulaire (16).
